**Europäisches Patentamt**

**European Patent Office**

**Office européen des brevets**

(19)

(11) Publication number : **0 364 100 B1**

## (12) EUROPEAN PATENT SPECIFICATION

(45) Date of publication of patent specification :
**25.11.92 Bulletin 92/48**

(51) Int. Cl.⁵ : **C07H 5/02,** C07H 13/04, C07H 13/08

(21) Application number : **89309227.0**

(22) Date of filing : **12.09.89**

(54) **Process for the chlorination of sugars.**

(30) Priority : **16.09.88 GB 8821804**
**27.09.88 GB 8822673**

(43) Date of publication of application :
**18.04.90 Bulletin 90/16**

(45) Publication of the grant of the patent :
**25.11.92 Bulletin 92/48**

(84) Designated Contracting States :
**AT BE CH DE ES FR GR IT LI LU NL SE**

(56) References cited :
**EP-A- 0 031 651**
**EP-A- 0 043 649**
**EP-A- 0 221 717**
**W.E. BACHMANN et al.: "Organic synthesis",
vol. 25, 1945, pages 84-85, John Wiley & Sons,
New York, US**

(73) Proprietor : **TATE & LYLE PUBLIC LIMITED
COMPANY
Sugar Quay Lower Thames Street
London, EC3R 6DQ (GB)**

(72) Inventor : **Khan, Riaz Ahmed
Rumsey Old Bath Road
Sonning Berkshire (GB)**
Inventor : **Sankey, George Henry
22 Falstaff Avenue
Early Reading Berkshire, RG6 2TQ (GB)**
Inventor : **Simpson, Philip John
65 Rowan Road
Tadley Hampshire, RG26 &RQ (GB)**
Inventor : **Vernon, Nicholas M.,
700 Fourth Street, M55
Athens Georgia 30601 (US)**

(74) Representative : **Ablewhite, Alan James et al
MARKS & CLERK 57/60 Lincoln's Inn Fields
London WC2A 3LS (GB)**

Note : Within nine months from the publication of the mention of the grant of the European patent, any
person may give notice to the European Patent Office of opposition to the European patent granted.
Notice of opposition shall be filed in a written reasoned statement. It shall not be deemed to have been
filed until the opposition fee has been paid (Art. 99(1) European patent convention).

## Description

This invention relates to the chlorination of sugars to produce chlorodeoxy derivatives, and in particular to the chlorination of sucrose derivatives in the preparation of sucralose (4,1',6'-trichloro-4,1',6'-trideoxy <u>galacto</u> sucrose).

There are a number of processes for the preparation of sucralose, all of which involve chlorination of the sucrose molecule in the 4-, 1'-, and 6'- positions. In order to achieve this, it is essential that the 6-position on the sucrose molecule is protected, since the 6-hydroxy group, being a primary hydroxy group, is highly reactive to chlorinating reagents. In the method of US 4380476 and GB 2079749 sucrose is acylated at the 6- position and the 4-, 1'- and 6'- positions are then chlorinated in the presence of unprotected hydroxy groups at the 2-, 3-, 3'- and 4'- positions.

A related process is disclosed in GB 2181734A. In this process, the 6- substituted sucrose is the trisaccharide raffinose which is a 6-α-D-galactopyranosyl derivative of sucrose. Its use in this process was predicated on the finding that it could be chlorinated to provide 6",4,1',6'-tetrachloro-6",4,1',6'-tetradeoxy<u>galacto</u>-raffinose, referred to for convenience as TCR. TCR can then be cleaved in the presence of a suitable α-galactosidase to yield sucralose.

An alternative method for preparing sucrose 6-esters is disclosed in copending US Application Serial No.220,641 filed 18th July 1988. Sucrose is reacted with a 1,3-dihydrocarbyloxy-1,1,3,3-tetra(hydrocarbyl)-distannoxane to produce a 1,3-di-(6-0-sucrose)-1,1,3,3-tetra(hydrocarbyl)-distannoxane, which can be regioselectively reacted with an acylating agent to produce the sucrose 6-acylate.

The method of chlorinating raffinose disclosed in GB 2181734A involves the use of thionyl chloride in pyridine in the presence of a triarylphosphine oxide or sulphide. Although this process gives the required chloro derivative, and in particular inserts a chlorine atom in the 4- position, it has considerable disadvantages. Firstly, the process employs 3 molar equivalents of the triarylphosphine oxide or sulphide, which is troublesome to remove and, if desired in the case of the oxide, to recycle. Secondly, the reaction conditions give rise to large amounts of black insoluble by-product, leading to difficulties in the work-up. The reaction also uses large quantities of thionyl chloride and yet the yields are only moderate.

In the chlorination of all 6- substituted sucrose derivatives, there is also the problem that it is not easy to obtain the correct degree of chlorination, ie. to chlorinate not only at the primary 6'-hydroxy groups, but also at the secondary (and somewhat sterically hindered) 4- position, and the primary 1'-position, yet not at the other positions.

We have now found that a modification of a long-known chlorination technique can be used to give the required chlorinated products in good yields.

Chlorination of alcohols using thionyl chloride and pyridine has been known for very many years (Darzens, Comptes Rendues, 1911, <u>152</u>, 1314, 1601; and 1912, <u>154</u>, 1615). The mechanism of the process was explained by Gerrard (Gerrard, J.Chem. Soc. 1939, 99; 1940, 218; and 1944, 85). In a first stage, two alcohol molecules ROH react with thionyl chloride to form a sulphite $R_2SO_3$ and two molecules of hydrogen chloride which react with the pyridine to form pyridine hydrochloride. In a second stage, the sulphite is decomposed by reaction with further thionyl chloride to provide two molecules of a chlorosulphite $RSO_2Cl$. In a third stage, the chlorosulphites react with pyridine hydrochloride to provide two molecules of chloride RCl and two molecules of sulphur dioxide.

Thus, in the Darzens process, pyridine acts as a solvent for the reactants, as an acid acceptor for the hydrogen chloride released during the initial reaction of thionyl chloride with the alcohol to form the sulphite and, in the form of pyridine hydrochloride, as a catalyst for the release of chloride ions for the last stage of the reaction. For polyhydroxy compounds where large quantities of hydrogen chloride are released the action of pyridine as an acid acceptor prevents degradation of the polysulphite.

When this process is applied to polyhydroxy compounds such as sugars, it might be expected that intramolecular sulphites are produced, and in practice the result is always an exceedingly complex mixture of products. It is presumably for this reason that there appears to be no published example of the thionyl chloride-pyridine reagent system being used successfully to chlorinate sugars. The nearest approach is seen in the process of GB 2 181 734 A, which uses triphenyl phosphine oxide in conjunction with thionyl chloride and pyridine to chlorinate raffinose but, as mentioned above, the results are far from satisfactory.

We have now found that sucrose, protected at the 6-position, or sucrose itself, can be reacted with thionyl chloride and a base such as pyridine or an alkyl-substituted pyridine to provide a good yield of the required chlorinated product, provided certain conditions are met.

Firstly, the amounts of thionyl chloride and of pyridine should be approximately 1 molar equivalent (ME) for every free hydroxyl group in the sugar molecule. Thus, a sucrose 6-ester, having 7 free hydroxyl groups (of which 3 are to be chlorinated) should be reacted with about 7 ME of thionyl chloride and about 7 ME of pyridine. Similarly, raffinose, having 11 free hydroxyl groups (of which 4 are to be chlorinated), should be reacted with about 11 ME of thionyl chloride and about 11 ME of pyridine.

In practice, the amount can vary to a certain extent. In general, for a sucrose derivative having $\underline{n}$ free hydroxyl groups, it is desirable to use from 0.9 $\underline{n}$ to 1.2 $\underline{n}$ ME of thionyl chloride and from $\underline{n}$ to 1.4 $\underline{n}$ ME of pyridine particularly $\underline{n}$ to 1.1 $\underline{n}$ of thionyl chloride and $\underline{n}$ to 1.3 $\underline{n}$ of pyridine.

When smaller amounts of pyridine are used the 6-protected sucrose intermediate does not dissolve completely, which can cause difficulties in stirring the reaction mixture initially, and there is insufficient base to neutralise the hydrogen chloride liberated during the first stage of the reaction. When larger amounts of pyridine are used, wasteful side reaction between thionyl chloride and the excess pyridine leads to the formation of undesirable by-products which are difficult to remove.

Secondly, the reaction should proceed in a non-reactive solvent in which the chlorosulphite is readily soluble, of moderate polarity e.g having a dielectric constant of from 5 to 15. Chlorinated hydrocarbons such as partially chlorinated ethanes are preferred solvents, with 1,1,2-trichloroethane being most preferred because it offers shorter reaction times (e.g. two hours or less at a reflux temperature of 112°). 1,2-Dichloroethane is lower boiling (reflux 83°; reaction time 9-12 h) and, hence, less preferred.

The reaction is advantageously effected by gradual addition of a solution of the sucrose derivative in pyridine to a solution of thionyl chloride in the chemically inert solvent. The reaction should initially take place at a lower temperature, e.g. at or below -5°C, or more preferably at about ambient temperature, followed by a period at an elevated temperature, conveniently the reflux temperature of the mixture when the reaction is effected under atmospheric pressure (about 83°C for 1,2-dichloroethane/pyridine and 112°C for 1,1,2- trichloroethane/pyridine).

Reference has been made to use of pyridine or alkyl-substituted pyridines in the reaction. We find that it is essential that an organic base is used. The base is necessary to neutralise hydrogen chloride which is evolved in the initial reaction of thionyl chloride with the hydroxyl group, which is believed to form an initial chlorosulphite and hydrogen chloride. In the absence of a base, the hydrogen chloride causes degradation of 6-substituted sucrose molecules, which are relatively acid-labile. It appears that an aromatic nitrogen base is the most suitable. Pyridine and alkyl-substituted pyridines are particularly suitable because they are good solvents for the sugar derivatives. Of the alkyl-substituted pyridines, 3-picoline and 4-picoline and mixtures of these two are the most suitable. In a second phase of the reaction the base hydrochloride acts as a source of chloride ions which displace the sulphite or chlorosulphite groups initially formed.

According to the present invention, therefore, we provide a process for chlorinating sucrose or a derivative thereof, particularly a 6- protected derivative such as a 6-ester or 6-ether, e.g. a glycosyl derivative such as raffinose, comprising reaction with thionyl chloride and a nitrogen base at a ratio of about one molar equivalent of thionyl chloride and about one molar equivalent of base for every molar equivalent of free hydroxyl in the sucrose or derivative thereof, in a non-reactive, moderately polar solvent. The method of the present invention provides an efficient and selective method of chlorinating sucrose 6-esters of use in the preparation of sucralose. The term '6-ester' also includes sucrose derivatives with a 6-ester group as well as an ester group elsewhere, for example a sucrose 6,4'-diester as described and claimed in GB 2 224 733.

Thus, according to a further feature of the present invention there is provided a method for the preparation of sucralose comprising reaction of a 6-protected sucrose derivative with a chlorinating agent, characterised in that chlorination is effected by reaction with about one molar equivalent of thionyl chloride and about about one molar equivalent of base for every molar equivalent of free hydroxyl in the sucrose derivative, in an inert, moderately polar, solvent.

The following examples illustrate the invention further. (Norit, Amberlite, Rad Pak, Duolite and PREP-PAK are Trade Marks)

Example 1: Chlorination of sucrose 6-acetate

A solution of crystalline sucrose 6-acetate (5 g; purity 79.8% see below) in pyridine (7.89 ml: approx. 7 ME) was added dropwise over a period of 30 minutes to thionyl chloride (7.09 ml: approx. 7 ME, see below) in 1,2-dichloroethane (25 ml), maintaining the temperature below -5°C. The mixture was allowed to warm to ambient, then heated over 1 hour to reflux (83°C). Complete dissolution of any precipitate occurred by 45°C. The solution was refluxed for 12 hours, then concentrated to half volume. The concentrate was added to a cold mixture of 0.880 ammonia (20 ml) and methanol (20 ml) and heated at 45°C for 45 minutes. The solution was concentrated to a thin syrup which was partitioned between butanone (50 ml) and saturated aqueous sodium chloride (50 ml). The aqueous layer was extracted with further butanone (50 ml). The combined organic phases were decolorised with activated carbon (Norit GB2), deionised using Duolite DMF ($H^+/OH^-$) ion-exchange resin and concentrated to dryness. Analysis of the residue by HPLC (using a liquid chromatograph with refractive index detector, fitted with a Steel Resolve C18 5 micron column and eluting with acetonitrile:water 28:72) indicated a conversion of sucrose 6-acetate into sucralose and sucralose 6-acetate combined of 72%.
Analysis of sucrose 6-acetate and calculation of $SOCl_2$ required.

| | % | $SOCl_2$ (ME) allowed | $SOCl_2$ (ml) required |
|---|---|---|---|
| sucrose 6-acetate | 79.8 | 7 | |
| sucrose 4-acetate | 3.9 | 7 | 5.57 |
| sucrose | 9.8 | 8 | 0.84 |
| methanol | 6.0 | 1 | 0.68 |
| | 99.5 | | 7.09 |

Example 2: Chlorination of raffinose

A solution of raffinose (10 g, anhydrous) in pyridine (17.6 ml: 11 ME) was added dropwise over 30 minutes to thionyl chloride (15.9 ml: 11 ME) in 1,2-dichloroethane (50 ml) at -5°C. The mixture was allowed to warm to ambient, then heated to reflux (83°C) over 1 hour. After refluxing for 9 hours, the solution was concentrated to half volume. The concentrate was added to a cold mixture of 0.880 ammonia (50 ml) and methanol (50 ml) and heated at 45°C for 1 hour. The solution was concentrated to a thin syrup which was partitioned between butanone (75 ml) and saturated aqueous sodium chloride (75 ml). The aqueous layer was extracted with further butanone (4x50 ml). The combined organic phases were decolorised with activated carbon (Norit GB2), deionised using Duolite DMF ($H^+/OH^-$) ion-exchange resin and concentrated to dryness. Analysis of the residue by HPLC (using a liquid chromatography with a PREP-PAK 500/C18 column and eluting with acetonitrile:water, 20:80) indicated a conversion of raffinose into tetrachlororaffinose of 58%.

Example 3 Chlorination of raffinose

The conditions of Example 2 were modified by replacing the 1,2-dichloroethane by 1,1,2-trichloroethane and refluxing at 112°C for 1.5 hours. The product was analysed by HPLC as described for Example 2. A 60% conversion of raffinose into tetrachlororaffinose was obtained.

Example 4: Preparation of Sucralose pentaacetate (TOSPA)

Sucrose 6-acetate (500 g; purity about 80%) was dissolved in pyridine (920 ml; 8.2 ME) at 60°C, cooled to ambient temperature and added to a stirred solution of thionyl chloride (730 ml; 7.2 ME) in 1,1,2-trichloroethane (TCE) (2000 ml) over 90 minutes keeping the temperature below 20°C. After the addition, the reaction solution was heated to reflux (112°C) over 2 hours and held at reflux for 90 minutes. The mixture was then cooled to below 20°C and a solution of ammonia (S.G. 0.880; 2000 ml) in water (1000 ml) was then added over 75 minutes keeping the temperature below 30°C. The mixture was then allowed to settle, the lower organic phase (about 3300 ml) was separated off, and the aqueous phase (2700 ml) was back-extracted with TCE (500 ml). The combined extracts were concentrated to a syrup at 55°C. Acetic anhydride (500 ml) and sodium acetate (50 g) were then added and the mixture was heated at 70°C for one hour after which toluene (2000 ml) was added. The mixture was then cooled to about 30°C and water (1000 ml) was added.
Crystallisation was allowed to proceed at 5°C for two hours and then the crude TOSPA was collected, washed with toluene (500 ml) and dried (fluid bed at ambient temperature). Yield: damp, about 610 g: dry, about 420 g; molar yield 56%. Assay 82.4% by HPLC using a liquid chromatograph fitted with a Rad Pak A column (C18) 5 microns and eluting with acetonitrile/methanol/water (3/3/4).

Example 5: Preparation of sucralose

A sample of TOSPA from Example 4 (50g) was taken up in methanol (125 ml) and sodium methoxide (0.5g) was added. The mixture was stirred at room temperature for 1.5 hours under vacuum. The resulting solution was neutralised by stirring with Amberlite IRC 50 (H+) resin (7.5 g) and then the resin was removed by filtration and washed with methanol (25 ml). The filtrate and wash were stirred with decolourising charcoal (2 g) and celite (2 g) for 15 minutes, then the solution was clarified by filtration and concentrated to a froth in vacuo. Crystalline sucralose was isolated by taking up the froth in ethyl acetate (100 ml), filtering, washing with ethyl acetate (25 ml) and drying in vacuo at 40°C for 12 hours. Yield 23.1 g (94%).

Example 6:

Chlorination of sucrose-6-benzoate with thionyl chloride and pyridine in 1,1,2-trichloroethane

A solution of sucrose-6-benzoate (10.0 g, 1.00 ME) in pyridine (16.3 ml, 9.00 ME) was added dropwise to a stirred, cooled solution of thionyl chloride (12.3 ml), 7.50 ME) in 1,1,2- trichloroethane (80 ml) at a rate sufficient to keep the temperature below 10°C. A white precipitate formed. The addition took 20 mins. The slurry was gently heated to reflux over 1 hr. By about 40°C the solids had all dissolved to give an orange solution. The mixture was refluxed (112°C) for 2 hours while being monitored by tlc (see below).

The mixture was cooled to ambient temperature and a solution of concentrated ammonium hydroxide (20 ml) in methanol (20 ml) was added slowly with cooling. An exothermic reaction raised the temperature to 60°C and the mixture was stirred at that temperature for 1 hr. Water (40 ml) was added and, after stirring at 60°C for 20 mins, the phases were separated. The organic phase was concentrated to dryness to give 12.9 g of sucralose-6-benzoate as a tan solid which was directly converted into sucralose (Example 7).

Tlc System

A sample of the reaction mixture (0.5 ml) was added to 1.0 ml of a solution consisting of 1:1 concentrated ammonium hydroxide/methanol, and the mixture stirred at 60°C for 30 mins. A sample (2μl) was spotted on a silica gel tlc plate and developed using 20:5:0.2 dichloromethane:methanol:acetic acid. Observation was by both UV light and spraying with 5% ethanolic sulphuric acid and charring.

Example 7

Preparation and isolation of sucralose from crude sucralose-6-benzoate

Crude sucralose-6-benzoate (12.9 g; from Example 6) was stirred with methanol (100 ml) containing sodium methoxide (0.40 g) at room temperature. After 1 hr a dark-brown clear solution had been formed, and after 2 hrs the reaction was complete by tlc (silica gel, eluant 20:5:0.2 dichloromethane:methanol:acetic acid). The pH was adjusted to 7.2 by stirring with 5.0 g of Amberlite IRC50H$^+$ ion exchange resin, and the resin filtered and washed with methanol. The filtrate was treated with activated carbon powder (1.0 g) and stirred at ambient temperature for 1 hr, filtered, and washed with methanol. The filtrate was concentrated to a brown oil (14.2 g).

The oil was submitted to an 8-stage counter-current liquid-liquid extraction process with partitioning between ethyl acetate (150 ml) and water (250 ml). The less polar impurities were extracted into the ethyl acetate, and the sucralose and more polar impurities were extracted into the aqueous phase. The aqueous phase was concentrated under reduced pressure to 25 ml and submitted to a 4-stage counter-current liquid-liquid extraction process with partitioning between 2-butanone 30 ml) and water (10 ml). The 2-butanone phases were concentrated to afford solid sucralose which was dried in vacuo. Yield 5.0 g. Assay: 92.4% sucralose, <2% chlorinated carbohydrate impurities.

HPLC Analytical Method

The sucralose sample was analyzed by high-performance liquid chromatography (HPLC). Sample components were separated on a reversed-phase, octadecylsilane HPLC column, using a mobile phase of 12% acetonitrile/88% water and a flow gradient increasing from 0.6 to 1.8 ml/min. Detection was by differential refractometry. The sample was analyzed against a known sucralose standard and standards of five impurities to determine percentage composition by weight.

Example 8

Chlorination of sucrose-6-benzoate with thionyl chloride and 3-picoline in 1,1,2-trichlorethane

A solution of sucrose-6-benzoate (10.0 g, 1.00 ME) in 3-picoline (17.6 ml, 8.0 ME) was added dropwise to a stirred, cooled solution of thionyl chloride (12.3 ml, 7.50 ME) in 1,1,2- trichloroethane (40 ml) at a rate sufficient to keep the temperature at 20 ± 2°C. A pale yellow turbid solution resulted. The mixture was heated slowly to reflux (110°C) over 1 hour and at reflux for a further hour. The reaction was monitored by tlc (see Example 5 for method).

The mixture (about 70 ml) was cooled to 30°C, transferred to a dropping funnel, and added dropwise, with stirring, to a saturated aqueous solution of ammonia (40 ml) with cooling. The addition took 10 mins maintaining the temperature below 30°C. The mixture was heated to 60°C for 20 mins, then the phases were separated. The organic phase was concentrated to dryness in high vacuum to afford 12.6 g of crude product shown by HPLC analysis to contain 57.4% sucralose-6-benzoate (63.3% yield).

HPLC Analytical Method

Sucralose-6-benzoate samples were analyzed by high-performance liquid chromatography (HPLC). Sample components were separated on a reversed-phase, octadecylsilane HPLC column, with gradient elution from 24% methanol/76% $0.01M$ $K_2HPO_4$, pH 7.5 buffer to 69.5% methanol/30.5% buffer. Detection was by ultraviolet absorption at 254 nm. Samples were analyzed against a sucralose-6-benzoate standard of known composition and purity to determine percentage by weight. Chromatographic purity was also calculated from the total chromatographic peak profile.

Reaction Profile

The graphs in Figure 1 represent a reaction profile of a $SOCl_2$-TCE-picoline chlorination of sucrose-6-benzoate. The reaction was conducted with 50.0 g (1.0 ME) of sucrose-6-benzoate, 88.0 ml (8.0 ME) of 3-picoline, 60.6 ml (7.5 ME) of $SOCl_2$, and 200 ml of TCE in accordance with the fundamentals of the procedure outlined above. Reaction aliquots were withdrawn at intervals and analyzed by HPLC for chlorodeoxysucrose derivatives. The graph shows that sucralose-6-benzoate ("tosben") formation is at its maximum after 1 hour at reflux when a 1 hour heat up period is employed. Extended heating results in a loss of sucralose-6-benzoate and the production of other chlorinated species.

Example 9

Chlorination of sucrose-6-benzoate with thionyl chloride and pyridine in 1,1,2-trichloroethane: 50 g scale isolating crystalline sucralose-6-benzoate

Sucrose-6-benzoate (50.0 g, 1.00 ME was dissolved in pyridine (72.5 ml, 8.00 ME) with warming. The solution was cooled to ambient temperature and added dropwise, with stirring, to a solution of thionyl chloride (60.6 ml, 7.50 ME) in 1,1,2-trichloroethane (200 ml) at a rate sufficient to maintain a temperature of 30°C, using ice-water cooling. The addition took 17 minutes. The resultant orange solution was heated linearly to 109°C over 40 minutes, gas evolution commencing at 90°C. The mixture was refluxed for 70 min, then cooled to 40°C.

The chlorination mixture (about 325 ml) was transferred to a dropping funnel and added dropwise to concentrated aqueous ammonia (190 ml) maintaining the temperature <30°C with ice cooling; the addition took 40 minutes. The biphasic mixture was heated at 60°C for 1 hour with vigorous stirring. The phases were separated and the aqueous phase was washed with 1,1,2-trichloroethane (25 ml). The combined organic phases were filtered to remove suspended solids and concentrated in vacuo to a froth. Yield 55.6 g, 58.3% from sucrose-6-benzoate corrected yield. Assay: 57.8% sucralose-6-benzoate, 5.8% dichlorinated sucrose-6-benzoates, and 16.3% tetrachlorinated sucrose-6-benzoates.

A portion of the crude product (25.0 g) was dissolved in dichloromethane (100 ml) and treated with activated carbon for 30 min at reflux. The solution was filtered through a bed of celite, washing the cake with dichloromethane (50 ml). The filtrate was concentrated to about 80 ml and was allowed to evaporate over 2 days standing at ambient conditions. The resulting gummy crystals were slurried in cold dichloromethane (40 ml) and filtered, washed with dichloromethane (20 ml), and dried. Yield 8.62 g. Assay: 89.5% sucralose-6-benzoate, 5.7% dichlorinated sucrose-6-benozates, and 4.0% tetrachlorinated sucrose-6-benzoate.

Example 10

Chlorination of sucrose-6-benzoate with thionyl chloride and pyridine in 1,2-dichloroethane

A solution of poor quality sucrose-6-benzoate (20.0 g, 1.00 ME; assay: 80.5% sucrose-6-benzoate, 18.5% sucrose) in pyridine (30.8 ml, 8.50 ME) was added dropwise to a cooled stirred solution of thionyl chloride (24.6 ml, 7.50 ME) in 1,2- dichloroethane (80 ml) over 20 minutes, maintaining the temperature less than 15°C. A thick white paste resulted which was allowed to warm to ambient temperature over 15 min, then gently heated to reflux over a further 30 min. Reflux was continued for 13 hr following the course of the reaction by tlc (see Example 5 for method).

The reaction mixture was cooled to 10°C and 1:1 conc. aqueous ammonia-methanol (80 ml) was added, maintaining the temperature below 30°C. The mixture was heated at 50°C for 1 hr and water (40 ml) was then added. The phases were separated and the aqueous phase was extracted with 1,2-dichloroethane at 50°C. The combined organic phases were extracted with water (40 ml), then concentrated to an oil (31.0 g) in vacuo.

The oil was converted to sucralose by deacylation in methanolic sodium methoxide, following standard procedures, to yield a brown froth containing 8.25 g sucralose, a 57.5% yield from sucrose-6-benzoate.

Example 11

Chlorination of sucrose with thionyl chloride and pyridine in 1,1,2-trichloroethane

An heterogeneous mixture of sucrose (1 g) and pyridine 2.3 ml, 10 ME) was treated with thionyl chloride (1.7 ml, 8 ME) in 1,1,2-trichloroethane (4 ml) at 0°C. The reaction was brought to ambient temperature and then heated at 95°C for 16 hr. The solution was neutralized with methanolic ammonia, concentrated to a syrup, and acetylated with acetic anhydride and pyridine at ambient temperature for 6 hr. The solution was concentrated, taken up in ether, washed with water, dried ($Na_2SO_4$), and concentrated to give a syrup (1.5 g). Glc analysis showed it to be a mixture of peracetates of 4,6,1',6'-tetrachloro-4,6,1',6'-tetradeoxy<u>galacto</u>sucrose (26.4%), 4,6,6'-trichloro-4,6,6'-trideoxy<u>galacto</u>sucrose (8.3%) and 6,6'-dichloro-6,6'-dideoxysucrose (17.0%).

Example 12

Chlorination of sucrose 6-acetate

Sucrose 6-acetate (500 g; purity about 80%) was dissolved in pyridine (950 ml) and the solution was added to a stirred solution of thionyl chloride (730 ml) in 1,1,2-trichloroethane (TCE, 2000 ml) over 90 minutes, keeping the temperature below 20°C. The reaction mixture was then heated to reflux over 2 hours and held at reflux (112°C) for 90 minutes. The mixture was then cooled to about 10°C and water (1000 ml) was added over 30 minutes, keeping the temperature below 20°C. A mixture of ammonia (S.G. 0.880; 1700 ml) and water (500 ml) was then added over 60 minutes, keeping the temperature below 30°C. The mixture was then allowed to settle, the organic phase was separated off and the aqueous phase was extracted with a mixture of TCE and pyridine (4:1, 500 ml). The combined organic extracts were concentrated at 55°C until about 1.5 litres of distillate had been collected and then cooled to about 20°C. Acetic anhydride (500 ml) was then added and the mixture was warmed to 60°C. More solvent (1000 ml) was then distilled off, xylene (2000 ml) was added and distillation was continued until a further 1000 ml of distillate had been removed. More xylene (1000 ml) was added, the mixture was cooled to about 25°C and water (1000 ml) was added. The mixture was then seeded and cooled to 5°C for 2 hours. The product was collected, washed with xylene (500 ml) and dried (fluid bed at 40°C). Yield 570 g (damp); 524 g (dry); molar yield 65%. Assay 78.5% (by HPLC as described in Example 4) with 13% xylene of crystallisation.

Comparative Example 1

Chlorination of sucrose 6-acetate with thionyl chloride and pyridine in 1,2-dichloroethane, using 9 ME thionyl chloride and 5 ME pyridine per ME sucrose 6-acetate

Sucrose 6-acetate (5g; purity about 80%) was taken up in pyridine (5.6 ml; 5ME) and added dropwise over a period of 30 minutes to a stirred solution of thionyl chloride (9.1 ml; 9ME) in 1,2-dichloroethane (25ml), maintaining the temperature at -5°C. The mixture was allowed to warm to ambient temperature, then heated over one hour to reflux (83°C). The solution was refluxed for 20 hours then concentrated to half volume. The concentrate was added to a cold mixture of ammonia (S.G. 0.880; 20 ml) and methanol (20 ml) and heated at 45°C for 45 minutes. The solution was then concentrated to a thin syrup and partitioned between butanone (50 ml) and saturated aqueous ammonium chloride (50 ml). The aqueous layer was extracted with further butanone (50 ml) and the organic phases were combined, decolorised with Duolite DMF (H$^+$/OH$^-$) ion-exchange resin and concentrated to dryness. Analysis of the residue by HPLC, using the method described in Example 1, showed a conversion of sucrose 6-acetate into sucralose and sucralose 6-acetate combined of about 5%.

Comparative Example 2

Chlorination of sucrose-6-benzoate with 1.07 ME thionyl chloride and 0.1 ME 3-picoline per ME hydroxyl in 1,1,2-trichloroethane:

Sucrose-6-benzoate (2.50 g, 0.10 ME) was dissolved in 3-picoline (4.40 ml, 0.8 ME) and added dropwise to a solution of thionyl chloride (30.3 ml, 7.50 ME) in 1,1,2-trichloroethane (100 ml) over 5 minutes at 15°C. Solid sucrose-6-benzoate (22.5 g, 0.90 ME, i.e a total of 1.0 ME of heptahydroxy material) was then added portionwise over 30 min to the solution at 15°C. No rise in temperature was observed. The sucrose-6-benzoate dissolved readily to give a clear, pale-yellow solution with copious gas emission. The mixture was heated to reflux (110°C) over 50 min and refluxed for a total of 7.2 hr, monitoring the course of the reaction by tlc (see Example 4 for method).

Considerable decomposition occurred and the reaction was stopped after 7.2 hr because of this.

The chlorination mixture was cooled to 20°C and conc. aqueous ammonia (100 ml) was added dropwise over 1 hr, cooling the mixture to 30°C. After stirring for a further 4 hr at room temperature, water (100 ml) was added and the phases were separated. The organic phase was concentrated in vacuo to a black oil (11.1 g) which contained 22.1% sucralose-6-benzoate, along with residual picoline and various degradation products.

## Claims

1. A process for the chlorination of sucrose or a derivative thereof, in which the sucrose or derivative thereof is reacted with thionyl chloride and a nitrogen base at a ratio of about 1 molar equivalent (ME) of thionyl chloride and about 1 ME of base for every ME of free hydroxyl, in a non-reactive moderately polar solvent.

2. A process according to claim 1 in which the ME ratio of thionyl chloride to free hydroxyl is from 0.9:1 to 1.2:1.

3. A process according to claim 2 in which the said ME ratio is from 1:1 to 1.1:1.

4. A process according to any of claims 1 to 3 in which the ME ratio of base to free hydroxyl is from 1:1 to 1.4:1.

5. A process according to claim 4 in which the said ratio is from 1:1 to 1.3:1.

6. A process according to any of claims 1 to 5 in which the nitrogen base is pyridine or an alkyl pyridine.

7. A process according to any of claims 1 to 6 in which the solvent is a chlorinated hydrocarbon.

8. A process according to claim 1 to 7 in which the solvent is a partially chlorinated ethane.

9. A process according to claim 8 in which the solvent is 1,1,2-trichloroethane.

10. A process according to any of claims 1 to 9 in which the sucrose derivative is a 6- protected sucrose.

11. A process according to claim 10, in which the 6-protected sucrose is a 6-ester or 6-ether, or a 6,4'-diester.

12. A process according to claim 11, in which the 6-protected sucrose is sucrose 6-acetate, sucrose 6-benzoate or raffinose.

13. A process for the preparation of sucralose comprising reaction of a 6- protected sucrose derivative with a chlorinating agent in the presence of a nitrogen base and subsequently removing the 6-substituent characterised in that the 6-substituted derivative is reacted with about one ME thionyl chloride and about one ME of base for every ME of free hydroxyl in the derivative.

EP 0 364 100 B1

**Patentansprüche**

1. Verfahren zur Chlorierung von Sucrose oder einem Derivat davon, wobei die Sucrose oder ein Derivat davon mit Thionylchlorid und einer Stickstoffbase in einem Verhältnis von etwa 1 molaren Äquivalent (ME) an Thionylchlorid und etwa 1 ME der Base für jedes ME an freiem Hydroxyl in einem nicht-reaktiven, schwach polaren Lösungsmittel zur Reaktion gebracht wird.

2. Verfahren nach Anspruch 1, dadurch **gekennzeichnet,** dass das ME-Verhältnis von Thionylchlorid zu dem freien Hydroxyl von 0,9:1 bis 1,2:1 ist.

3. Verfahren nach Anspruch 2, dadurch **gekennzeichnet,** dass das ME-Verhältnis von 1:1 bis 1,1:1 ist.

4. Verfahren nach einem der Ansprüche 1 bis 3, dadurch **gekennzeichnet,** dass das ME-Verhältnis von Base zu freiem Hydroxyl von 1:1 bis 1,4:1 ist.

5. Verfahren nach Anspruch 4, dadurch **gekennzeichnet,** dass das Verhältnis von 1:1 bis 1,3:1 ist.

6. Verfahren nach einem der Ansprüche 1 bis 5, dadurch **gekennzeichnet,** dass die Stickstoffbase Pyridin oder ein Alkylpyridin ist.

7. Verfahren nach einem der Ansprüche 1 bis 6, dadurch **gekennzeichnet,** dass das Lösungsmittel ein chlorierter Kohlenwasserstoff ist.

8. Verfahren nach Anspruch 1 bis 7, dadurch **gekennzeichnet,** dass das Lösungsmittel ein teilweise chloriertes Ethan ist.

9. Verfahren nach Anspruch 8, dadurch **gekennzeichnet,** dass das Lösungsmittel 1,1,2-Trichlorethan ist.

10. Verfahren nach einem der Ansprüche 1 bis 9, dadurch **gekennzeichnet,** dass das Sucrosederivat eine 6-geschützte Sucrose ist.

11. Verfahren nach Anspruch 10, dadurch **gekennzeichnet,** dass die 6-geschützte Sucrose ein 6-Ester oder 6-Ether oder ein 6,4′-Diester ist.

12. Verfahren nach Anspruch 11, dadurch **gekennzeichnet,** dass die 6-geschützte Sucrose Sucrose-6-acetat, Sucrose-6-benzoat oder Raffinose ist.

13. Verfahren zur Herstellung von Sucralose, umfassend die Reaktion eines 6-geschützten Sucrosederivates mit einem Chlorierungsmittel in Gegenwart einer Stickstoffbase und die anschliessende Entfernung des 6-Substituenten, dadurch **gekennzeichnet,** dass das 6-substituierte Derivat mit etwa 1 ME Thionylchlorid und etwa 1 ME Base für jedes ME an freiem Hydroxyl in dem Derivat zur Reaktion gebracht wird.

**Revendications**

1. Un procédé pour la chloruration du saccharose ou d'un de ses dérivés, dans lequel on fait réagir le saccharose ou son dérivé avec du chlorure de thionyle et une base azotée dans un rapport d'environ 1 équivalent molaire (ME) de chlorure de thionyle et environ 1 ME de base pour chaque ME de groupement hydroxyle libre, dans un solvant non-réactif modérément polaire.

2. Un procédé suivant la revendication 1, dans lequel le rapport ME de chlorure de thionyle sur le groupement hydroxyle libre est compris entre 0.9:1 et 1.2:1.

3. Un procédé suivant la revendication 2, dans lequel ledit rapport ME est compris entre 1:1 et 1.1:1.

4. Un procédé suivant l'une quelconque des revendications 1 à 3, dans lequel ledit rapport ME de la base sur le groupement hydroxyle est compris entre 1:1 et 1.4:1.

5. Un procédé suivant la revendication 4, dans lequel ledit rapport ME est compris entre 1:1 et 1,3:1.

6. Un procédé suivant l'une quelconque des revendications 1 à 5, dans lequel la base azotée est la pyridine ou une alkyl pyridine.

7. Un procédé suivant l'une quelconque des revendications 1 à 6, dans lequel le solvant est un hydrocarbure chloré.

8. Un procédé suivant les revendications 1 à 7, dans lequel le solvant est l'éthane partiellement chloré.

9. Un procédé suivant la revendication 8, dans lequel le solvant est le 1,1,2-trichloroéthane.

10. Un procédé suivant l'une quelconque des revendications 1 à 9, dans lequel le dérivé de saccharose est un saccharose protégé en position 6.

11. Un procédé suivant la revendication 10, dans lequel le saccharose protégé en position 6 est un 6-ester ou un 6-éther ou un 6,4'-diester.

12. Un procédé suivant la revendication 11, dans lequel le saccharose protégé en position 6 est le 6-acétate de saccharose, le 6-benzoate de saccharose ou le raffinose.

13. Un procédé pour la préparation de sucralose qui comprend la réaction d'un dérivé de saccharose protégé en position 6 avec un agent de chloruration en présence d'une base azotée et l'enlèvement subséquent du substituant en position 6, caractérisé en ce qu'on fait réagir le dérivé substitue en position 6 avec environ un ME de chlorure de thionyle et environ un ME de base pour chaque ME de groupement hydroxyle libre dans le dérivé.